# EUROPÄISCHE PATENTANMELDUNG

(11) **EP 2 113 557 A2**
(43) Veröffentlichungstag der Anmeldung: **04.11.2009**
(21) Anmeldenummer: 09005143.4
(22) Anmeldetag: 08.04.2009
(51) Int. Cl.: C12M 1/12

(54) **In-situ Reinigung von im Bioreaktor integrierten Membranen**

(30) Priorität: 29.04.2008 DE 102008021330
(71) Anmelder: Chmiel, Prof. Dr.-Ing. habil., Horst, 80689 München (DE)
(72) Erfinder: Chmiel, Prof. Dr.-Ing. habil., Horst, 80689 München (DE)
(74) Vertreter: Flosdorff, Jürgen

(57) **Zusammenfassung**

Die Erfindung betrifft ein Verfahren und eine Vorrichtung zum kontinuierlichen Austrag von Produkt aus einem Bioreaktor mittels mehrerer parallel geschalteter intern oder extern im Kreislauf integrierter Membranmodule bei gleichzeitiger in-situ-Reinigung der Membranen. Dabei wird das für die Reinigung benötigte Fluid so gewählt, dass es nach der Reinigung in den Bioreaktor eingeleitet werden kann.

## Beschreibung

Die Erfindung betrifft ein Verfahren und eine Vorrichtung zur in-situ Reinigung von im Bioreaktor integrierten Membranen bei gleichzeitigem Produktaustrag.

Die Kosten von biotechnologisch gewonnenen Produkten werden wesentlich von der Produktisolation und Produktreinigung bestimmt.

Damit dies möglichst zeitnah geschieht wird angestrebt, diese Schritte in den Produktionsprozess zu integrieren und dabei gleichzeitig die Biomassekonzentration im Bioreaktor zu erhöhen. Prädestiniert hierfür sind poröse Membranen. Mikro-, Ultra- und Nanofiltrafionsmembranen sind bekanntlich poröse, flächige Gebilde, die Flüssigkeiten wie z.B. Wasser permeieren lassen, während die darin suspendierten oder gelösten Stoffe zurückgehalten werden. Für deren Integration in den Bioreaktor bieten sich hauptsächlich zwei Varianten an.

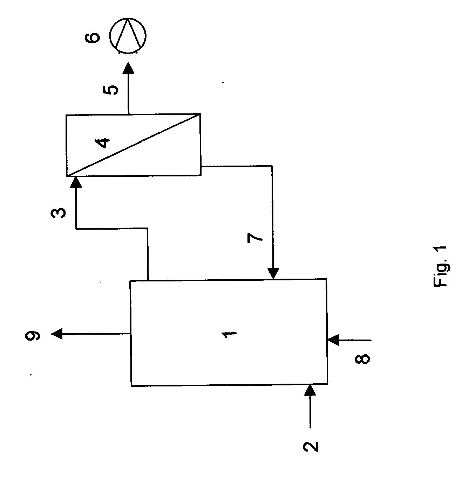

In Figur 1 ist die externe Integration von Membranen dargestellt. Dabei ist 1 der Bioreaktor, 2 das für die Produktion benötigte - dem Bioreaktor zugeführte - Medium, 3 das in der Regel ideale Gemisch aus Mikroorganismen, Medium und

Produkt, 4 der Membranmodul, in dem die Membranen fixiert sind, 5 das durch eine Vakuumpumpe 6 über die Membranen abgetrennte Produkt , 7 das Konzentrat an Mikroorganismen, das in den Bioreaktor zurück geführt wird, 8 ein Gas, das bei aeroben Prozessen aus Luft oder Sauerstoff besteht und in den Bioreaktor eingeblasen wird und schließlich 9 das Abgas.

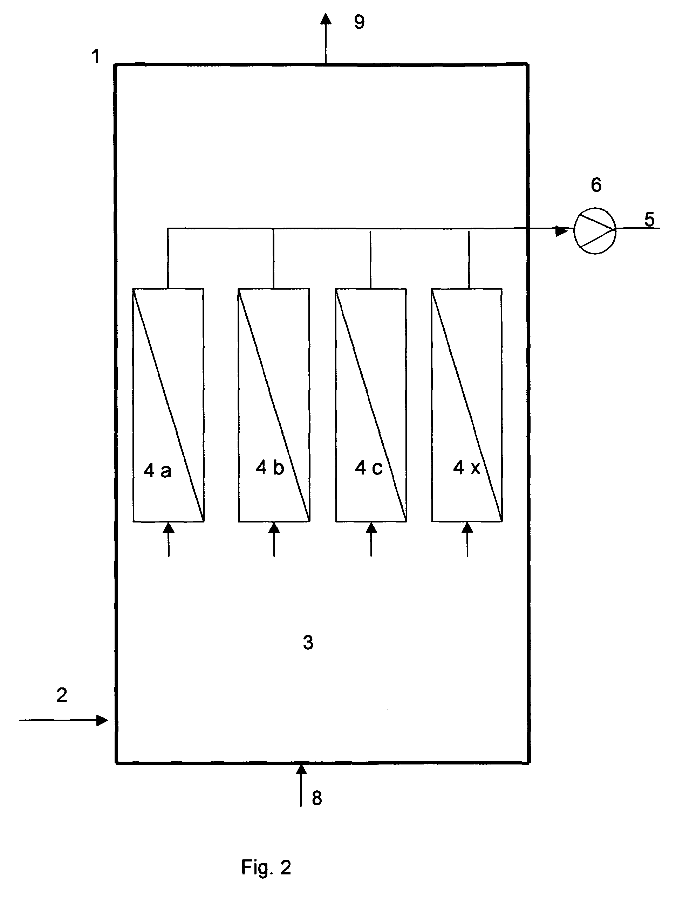

Die Variante zum externen Kreislauf ist die in Figur 2 dargestellte Installation der Membranen unmittelbar in den Bioreaktor (in das Medium getaucht).

Ein weiterer Vorteil der integrierten Membranen besteht darin, dass der Bioprozess kontinuierlich oder quasi kontinuierlich geführt werden kann. Das setzt allerdings voraus, dass die Membranen während der gesamten Produktionsdauer ihre Funktionsfähigkeit behalten. Leider trifft das in der Realität nicht zu. Vielmehr nimmt die Menge der die Membran bei konstanter transmembraner Druckdifferenz permeierenden Flüssigkeit, der so genannte Flux mit zunehmender Betriebsdauer immer mehr ab. Außerdem ändern sich die Trenneigenschaften der Membran, die Poren werden immer kleiner, es werden Moleküle zurück gehalten, die die Membran eigentlich permeieren sollen.

Ursache sind organische Ablagerungen auf der Membran und in den Membranporen, das so genannte Membranfouling. Als Gegenmaßnahme wird die Membran mit hoher Geschwindigkeit tangential angeströmt.

In Anordnung nach Figur 1 wird beispielsweise die Strömungsgeschwindigkeit durch den Modul erhöht. In Anordnung nach Figur 2 vergrößert man die eingetragene Gasmenge über den eigentlichen Sauerstoffbedarf der Mikroorganismen hinaus. In beiden Fällen bedeutet das eine Erhöhung des Energiebedarfs und eine Scherbeanspruchung der Mikroorganismen, die deren Produktivität mindert. Außerdem lassen sich mit dieser Maßnahme nur die Ablagerungen auf der Oberfläche der Membran, nicht aber das in den Membranporen adsorbierte Material entfernen, weshalb die Wirkung begrenzt bleibt. In der Literatur (Chmiel "Bioprozesstechnik", Elsevier-Verlag 2005) wird deshalb ein zyklisches Rückspülen der Membran mit Permeat, d.h. eine Umkehr der Förderrichtung der Pumpe 6 vorgeschlagen.

Das hat allerdings Nachteile:
1. Wird der Produktaustrag während des Rückspülvorgangs unterbrochen.
2. Wird ein beträchtlicher Teil des Permeats für die Rückspülung benötigt und muss daher bei der Berechnung des effektiven Membranflusses abgezogen werden.
3. Lässt sich der fest in der Pore adsorbierte Teil des organischen Materials nicht durch Permeatrückspülung entfernen.

Abhilfe würde hier nur eine vollständige Reinigung unter Einsatz von Chemikalien bringen. Dazu muss aber normaler Weise die Anlage komplett abgeschaltet werden, um zu verhindern, dass Reinigungsmittel in den Bioreaktor gelangen und die im Reaktor integrierten Membranen müssen zuvor aus dem Rektor entfernt werden, damit die Reinigungschemikalien nicht in den Bioreaktor gelangen und dort die Mikroorganismen schädigen oder das Produkt verunreinigen.

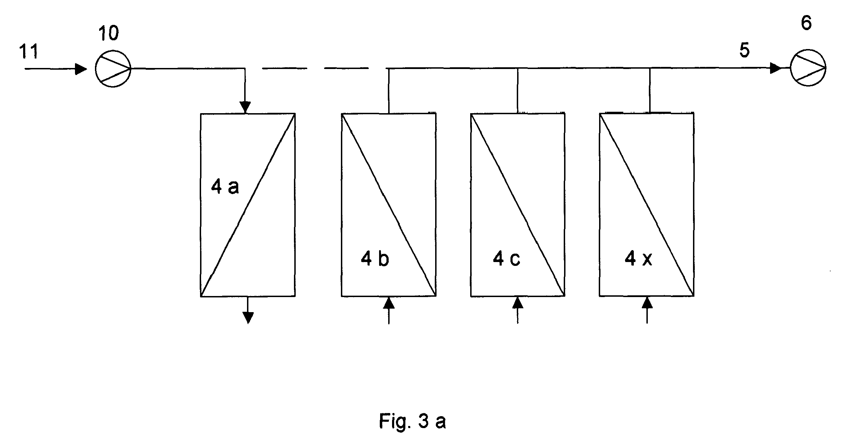

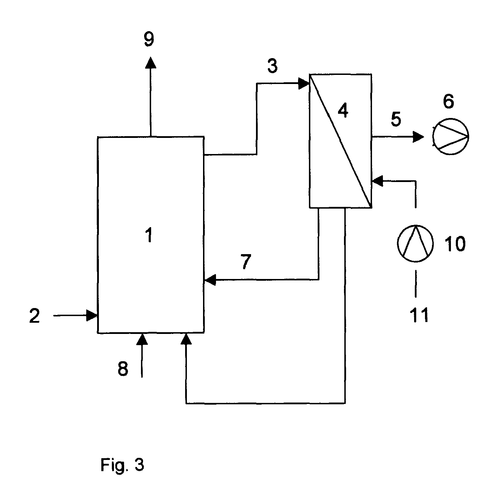

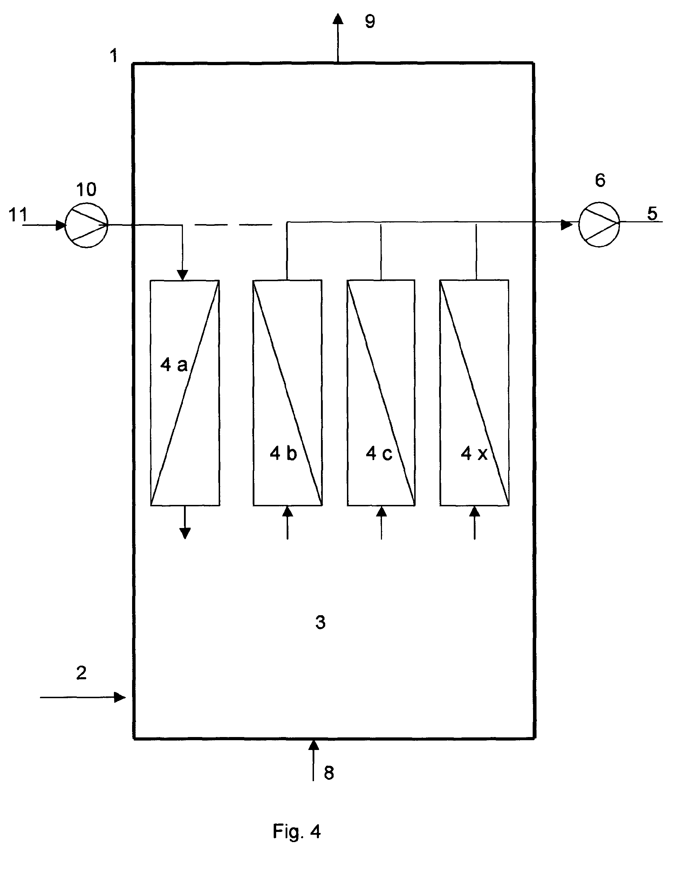

Die oben genannten Nachteile werden durch die Erfindung beseitigt. Sie wird durch die Figuren 3 und 4 erläutert.

Grundgedanke ist, dass als Reinigungschemikalie 11 ein Medium verwendet wird, das ohnehin der Anlage (dem Bioreaktor) zugeführt werden muss oder zugeführt werden darf. Das kann z.B. eine Base, eine Säure oder eine oxidierende Flüssigkeit, wie Wasserstoffperoxid oder Ozon sein, die gemäß Figuren 3 und 4 über die Pumpe 10 in zur Produktentfernung umgekehrter Richtung in einen von mehreren parallel geschalteten Membranmodulen (Modul 4a in Figur 3a) und von dort in den Bioreaktor gepumpt wird. Dieser befindet sich in Reinigungsposition, während die übrigen Module im Filtrationsmodus arbeiten (4 b bis 4 x). Durch geeignete Ventilsteuerung werden nun nacheinander die Module 4b bis 4x gereinigt, während die übrigen Module filtrieren, d. h. kontinuierlich Produkt aus dem Bioreaktor austragen. Das Ergebnis ist, dass Produktgewinnung und Membranreinigung gleichzeitig und in der Anlage geschehen. Man bezeichnet eine solche Reinigung auch als in-situ, d.h. am Ort geschehend.

Die Erfindung soll im Folgenden am Beispiel eines Anaerobreaktors zur Reinigung von Abwasser erläutert werden.

Die Reinigung hoch belasteter Abwässer mittels Anaerobtechnik gewinnt zunehmend an Bedeutung. Der wichtigste Grund ist sicherlich der, dass man damit Energie erzeugen kann, so dass der Reinigungsprozess energieneutral oder sogar mit einem Energieüberschuss erfolgt. Man rechnet mit ca. 0,44m³ Methan/kg CSB (COD) der umgesetzt wurde. Im Gegensatz hierzu muss bei der aeroben Wasserreinigung für die benötigte Sauerstoffzufuhr erheblich Energie zugeführt werden.

Ein weiterer Vorteil der anaeroben gegenüber der aeroben Abwasserreinigung ist, dass nur etwa 20 % Überschussschlamm entsteht, verglichen mit derjenigen der aeroben Abwasserreinigung.

Diesen Vorteilen stehen allerdings auch Nachteile gegenüber.

Der wichtigste ist die langsame Reinigung, d.h. die lange Verweilzeit des Abwassers im Bioreaktor.

Bekanntlich geschieht der anaerobe Abbau der Verunreinigungen in mindestens zwei Stufen:
- Umwandlung in organische Säuren (acidogene Stufe)
- Umwandlung der organischen Säuren in Gase, vor allem Methan, Kohlendioxid und Wasserstoff (methanogene Stufe)

Sowohl die acetogenen, insbesondere aber die methagenen Bakterien wachsen wesentlich langsamer (lange Verdopplungszeit) verglichen mit den aeroben Bakterien.

Ein weiterer Nachteil besteht in dem großen Bedarf an Chemikalien (so genannte Korrekturmittel), insbesondere um den pH-Wert in der acidogenen Stufe konstant zu halten. So muss z.B. eine beträchtliche Menge an Base (z.B. 0,2 bis 0,3 kg Natronlauge pro kg CSB) zugeführt werden, um den pH-Sollwert auf 6,2 zu halten.

Der erstgenannte Nachteil wird noch dadurch verstärkt, dass die methanogenen Bakterien mit dem gereinigten Wasser ausgewaschen werden.

In der Literatur werden daher Verfahren beschrieben, die Mikroorganismen daran hindem, den Reaktor zu verlassen. Prädestiniert hierfür wären die eingangs erwähnten poröse Membranen.

In einem Übersichtsreferat (Bao-Qiang Liao et. Al. Critical Reviews in Enviromental Science and Technology, 36: 489-530, 2006) wird allerdings festgestellt, dass anaerobe Mikroorganismen besonders gern auf Oberflächen aufwachsen. Im anaeroben Abwasserprozess integrierte Membranen neigen daher besonders stark zum Fouling. In der genannten Publikation werden die beiden in Figur 1 und Figur 2 dargestellten konstruktiven Lösungen mit integrierten Mikro- und

Ultrafiltrationsmembranen diskutiert.

Bei der ersten Lösung wird an den anaeroben Abwasserreaktor der Membranmodul in einem externen Kreislauf angeschlossen. Der gesamte Reaktorinhalt, also Abwasser samt Mikroorganismen, wird mit hoher Strömungsgeschwindigkeit ( bis zu 4,5 m/s) durch den Membranmodul gepumpt. Das bedeutet natürlich einen hohen Energieaufwand. Trotzdem geht der Fluss durch die Membran während der Betriebsdauer im Durchschnitt auf die Hälfte zurück. Außerdem verschlechtert sich die Reinigungsleistung im Anaerobreaktor, was als Folge der gestressten Mikroorganismen gesehen wird.

Bei der zweiten Lösung werden die Membranen im externen Kreislauf nicht durchströmt, sondern sie werden analog zu Figur 2 getaucht (submersed membranes) und mit Gas (z. B. Stickstoff) angeströmt. Das Permeat, also das gereinigte Wasser, wird durch Vakuum abgesaugt. Auch hier ist also Energie für die Anströmung der Membran mit Gas notwendig. Trotzdem geht der Fluss durch die Membran mit zunehmender Betriebsdauer noch stärker zurück, als bei der ersten Lösung.

Die Ursachen hierfür wurden ja bereits eingangs diskutiert.

Abhilfe würde hier nur eine vollständige Reinigung unter Einsatz von Chemikalien bringen. Dazu muss aber normaler Weise die Anlage komplett abgeschaltet und die Membranen aus dem Reaktor entfernt werden, um zu verhindern, dass Reinigungsmittel in den Bioreaktor gelangen.

Diese Nachteile werden durch die Erfindung beseitigt. Sie wurde bereits durch die Figuren 3 und 4 und hier ergänzend mittels Figur 5 erläutert.

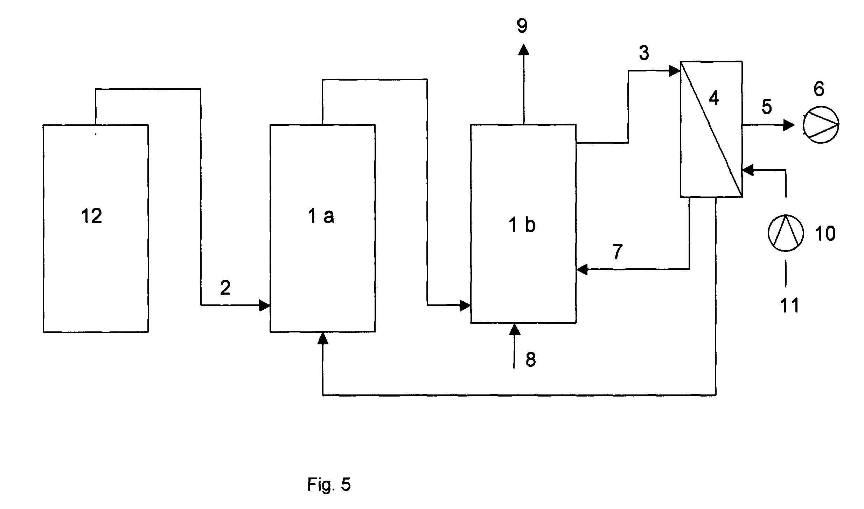

Figur 5 zeigt eine zweistufige anaerobe Abwasseranlage, d. h. der Bioreator besteht aus den Behältern 1a (acetogene Stufe) und 1b (methanogeneStufe), sowie einem Abwasserspeicherbehälter 12, wobei im externen Kreislauf eine Gruppe parallel geschalteter Membranmodule integriert ist. Gemäß Figur 3a befindet sich Modul 4a im Reinigungszustand, während die Module 4b bis 4x im Filtrationsmodus arbeiten.

In der Getränkeindustrie wird als Reinigungsmittel häufig Natronlauge verwendet. Andererseits wird Natronlauge auch zur pH-Regulierung an anaeroben Abwasseranlagen eingesetzt.

Gemäß Erfindung wird Membranmodul 4a von Natronlauge durchströmt, die anschließend gemäß Figur 5 in die acetogene Stufe (1a) der anaeroben Abwasseranlage geleitet wird. Dabei richtet sich die Menge an Natronlauge nach dem Bedarf zur pH-Stabilisierung der acetogenen Stufe. Da diese, verglichen mit der Permeatmenge der im Filtrationsmodus befindlichen Module, gering ist, kann man die Durchströmung des Moduls 4a als diffusiv bezeichnen d.h. es ist nur eine kleine Pumpe mit vernachlässigbarem Energiebedarf erforderlich.

Die erfindungsgemäße Besonderheit ist, dass der Betrieb der Anlage dadurch nicht gestört wird. Trotz der sukzessiven Reinigung der Membranen läuft der anaerobe Abwasserreinigungsprozess kontinuierlich und ungestört weiter.

## Patentansprüche

1. Verfahren zum kontinuierlichen Austrag von Produkt aus einem Bioreaktormittels poröser Membranen,
**dadurch gekennzeichnet,**
**dass** über mehrere parallel durchströmte Membranmodule mittels einer Pumpe aus dem Gemisch des Reaktorinhalts das Produkt ausgetragen wird, während gleichzeitig wenigstens einer dieser Membranmodule gereinigt wird, in dem er mittels einer zweiten Pumpe in zum Produktaustrag umgekehrter Richtung von einem Reinigungsfluid durchströmt wird und dieses in den Bioreaktor eingetragen wird.

2. Verfahren nach Anspruch 1, **dadurch gekennzeichnet, dass** das Reinigungsfluid eine Base, Säure, oxidierende Flüssigkeit oder eine Kombination hiervon ist.

3. Verfahren nach Ansprüchen 1 und 2 **dadurch gekennzeichnet, dass** das Reinigungsfluid Natronlauge ist.

4. Verfahren nach Ansprüchen 1 und 2, **dadurch gekennzeichnet, dass** das Reinigungsfluid Phosphorsäure ist.

5. Verfahren nach Ansprüchen 1 und 2, **dadurch gekennzeichnet, dass** das Reinigungsfluid H2O2 ist .

6. Verfahren nach Ansprüchen 1 und 2, **dadurch gekennzeichnet, dass** das Reinigungsfluid Ozon ist.

7. Vorrichtung zum kontinuierlichen Austrag von Produkt aus einem Bioreaktor mit mehreren parallel durchströmbaren im Bioreaktor integrierten Membranmodulen und einer Pumpe zum Austrag des Produkts, **dadurch gekennzeichnet, dass** eine zweite Pumpe zum Eintrag eines Reinigungsfluids über jeweils wenigstens einen der Membranmodule in den Bioreaktor vorhanden ist.

8. Vorrichtung nach Anspruch 7, **dadurch gekennzeichnet, dass** der Bioreaktor ein Anaerobreaktor ist, in den die Membranmodule getaucht werden, das Medium Abwasser und das Produkt gereinigtes Wasser ist.

9. Vorrichtung nach Anspruch 7, **dadurch gekennzeichnet, dass** der Bioreaktor ein zweistufiger Anaerobreaktor ist, die Membranmodule in einem externen Kreislauf integriert sind, sowie das Medium Abwasser und das Produkt gereinigtes Wasser sind.
